# EUROPEAN PATENT SPECIFICATION

(11) **EP 3 625 167 B1**
(45) Date of publication and mention of the grant of the patent: **07.06.2023**
(21) Application number: 18731175.8
(22) Date of filing: 15.05.2018
(51) Int. Cl.: B82Y 30/00, G01N 31/22, G01N 21/78, G01N 33/18

(54) **A METHOD FOR DETECTING THE PRESENCE OF MERCURY IN WATER, AND A KIT FOR CARRYING OUT THE METHOD**
METHODE ZUR ANZEIGE DER PRÄSENZ VON QUECKSILBER IN WASSER UND KIT ZUR UMSETZUNG DER METHODE
PROCEDE D'AFFICHAGE DE LA PRESENCE DE MERCURE DANS L'EAU ET KIT DE MISE EN OEUVRE DU PROCEDE

(30) Priority: 15.05.2017 IT 201700052359
(43) Date of publication of application: 25.03.2020
(73) Proprietor: Fondazione Istituto Italiano di Tecnologia, 16163 Genova (IT)
(72) Inventor: BERTOLACCI, Laura, 16133 Genova (IT); POMPA, Pier Paolo, 73100 Lecce (IT)
(74) Representative: Palladino, Saverio Massimo
(86) International application number: PCT/IB2018/053380
(87) International publication number: WO 2018/211415

(56) References cited:
- CN-A- 104 155 252
- CN-A- 104 267 026
- US-A1- 2007 122 833
- YI JUAN LONG ET AL: "Visual observation of the mercury-stimulated peroxidase mimetic activity of gold nanoparticles", CHEMICAL COMMUNICATIONS, vol. 47, no. 43, 1 January 2011 (2011-01-01), pages 11939-11941, XP055438470, ISSN: 1359-7345, DOI: 10.1039/c1cc14294a

## Description

### FIELD OF THE INVENTION

The present invention refers to a method for colorimetric determination of the presence of mercury in samples of water, as well as a kit containing the reagents for carrying out the method.

### STATE OF THE ART

The presence of heavy metals in environmental matrices, such as soil and water, is an unavoidable consequence of various human activities. Although certain enzymes that are essential for cellular processes need, for their proper functioning, specific metal cations (defined as essential metals, such as for example Fe²⁺, Zn²⁺, Cu²⁺, Co²⁺, Ni²⁺, etc.), high doses of metals may exert a toxic action on both plant and animal organisms, first and foremost human beings. High toxicity is shared, in particular, by arsenic, cadmium, chromium, mercury and lead, also classified by the International Agency for Research on Cancer (IARC) as known or potential carcinogens. In particular, mercury gives rise to many concerns due to the consequences that a long exposure may cause as a damage to various organs, such as kidneys, lungs, and central nervous system. The limit of tolerability defined by the World Health Organization is 30 nM in drinking water; the European Union has lowered this limit to 5 nM.

Today, the monitoring of any mercury or other heavy metal contamination is carried out using analytical techniques, such as atomic absorption spectroscopy (AAS) or atomic emission (EAS), and inductively coupled plasma mass spectrometry (ICP-MS). However, these techniques, in addition to being expensive, require dedicated laboratories and qualified personnel, and the transport of samples from the sampling sites to the laboratories, with the consequence that the results of the analyses are available only with a delay, often days, with respect to the sampling.

It would instead be preferable to have methods available that allow the analysis of water samples and the detection of toxic metals directly on the sampling site, quickly and preferably at reduced costs; similar methods would open the way to numerous applications in scenarios ranging from the domestic environment to sites of suspected spills or, moreover, in developing countries.

For this reason, in recent decades, numerous attempts have been made to develop innovative techniques for the determination of heavy metals; the most promising in this direction are undoubtedly those that allow a colorimetric readout with the naked eye.

Various approaches have been followed for research purposes.

A first approach involves the use of enzymes that convert a chromogenic substrate, or generate a change in pH, resulting in the color change of an indicator. The mechanism of detection exploits the very nature of the analytes, which occupy the enzymatic pockets by replacing the constituent metals, and thus determining the inhibition of the enzyme. The use of enzymes ensures a high sensitivity, thanks to the amplification of the signal due to their turn over.

A second path followed involves the use of metal nanoparticle suspensions. In these suspensions, the chromatic variation can be induced through modifications of their surface or state of aggregation. This path has been widely explored, combining gold (GNPs) or silver (AgNPs) nanoparticles with the use of oligonucleotides (called aptamers) that can be covalently bonded or simply adsorbed on the nanoparticles surface; this approach is used e.g. in CN 104 155 252 A; peculiarity of these oligonucleotides is to be designed to recognize and bind the analytes to be determined.

A limitation of both these approaches is that the use of biomolecules, whether enzymes and/or aptamers, often implies the need to maintain salinity and pH ranges not too far from the physiological ones, both to prevent enzyme denaturation and to guarantee the functioning of the mechanisms of recognition and interaction between the biomolecules themselves and the analytes. This may be incompatible with other analysis conditions (e.g. analyte solubility, temperature, etc.). Moreover, in these methods it is often difficult to eliminate the interference caused by the macroelements in the matrix, such as Na⁺, K+, Mg²⁺ and Ca²⁺, due to the combination of two factors: their high affinity for biological molecules and their presence in concentrations higher than ppm, therefore approximately three orders of magnitude higher than the analytes to be determined.

A third approach is based on the exploitation of the catalytic properties of certain metal nanoparticles. This path combines the advantages of "enzymatic" signal amplification with nanoparticles long-term stability and ease of handling. In fact, various noble metals historically used as catalysts in chemical synthesis show, when they are in colloidal state, the same catalytic properties, further amplified by the extremely high surface/volume ratio. In particular, it has been widely demonstrated that gold and platinum nanoparticles (PtNPs) are able to mimic the peroxidase enzymatic action, that is to catalyze the redox reaction between hydrogen peroxide and oxidizable substrates. Due to this their ability, GNPs and PtNPs may act as "nanoenzymes" (name by which these particles are usually referred to in the field) by converting chromogenic substrates, as previously described in the case of enzymes.

Unlike enzymes, whose activity is concentrated in limited areas of the molecule and can be inhibited by a single analyte ion, the atoms of the entire nanoparticles surface can exert their catalytic activity, therefore the inhibition of nanoenzymes requires passivation of a considerable percentage of their surface.

Several methods are described in literature to selectively passivate nanoparticles according to the presence of an analyte; in particular, those involving a direct nanoparticle/analyte interaction result to be very interesting.

For example, some methods for the determination of Hg²⁺ involving a cation reduction step by the citrate ion present on the metal nanoparticle surface were published. The reduced analyte determines, through the formation of an amalgam on the nanoparticle surface, a variation in their peroxidase properties (increase or reduction, depending on the mechanism) with consequent increase or decrease in the generation of color that allows to detect the presence of analyte. The identification mechanism, which provides for the reduction of the analyte, is highly selective as it eliminates all interferences due to macroelements (in particular Na⁺, K⁺, Ca²⁺, Mg²⁺) present in high concentrations in drinking water and to elements generally present in traces, such as Mn²⁺, Fe²⁺, Zn²⁺, Co²⁺, Cu²⁺ and other possible contaminants, such as Ni²⁺, Cd²⁺, As³⁺, Pb²⁺ and Cr³⁺ because they are all characterized by redox potentials significantly lower than Hg²⁺.

An example of this approach is represented by the method of the patent application CN 104155252 A, wherein gold nanoparticles functionalized with single-stranded nucleic acids able to increase, once bound to the analyte, their peroxidase activity are used. This catalytic activity is detected through a detection system involving the use of H₂O₂ and the chromogenic substrate 3,3',5,5'-tetramethylbenzidine (TMB) which assumes a characteristic light blue color when oxidized.

The article "A simple paper-based colorimetric device for rapid mercury(II) assay", W. Chen et al., Scientific Reports 6, 31948 (2016), describes a paper-based colorimetric device for *in situ* analysis of mercury (II) in water. The operating principle of this device is based on the specific interaction between Pt nanoparticles and mercury ions and the ability of Hg-Pt amalgams to weaken the peroxidase catalytic activity of nanoparticles. The procedure consists in distributing a solution of TMB/H₂O₂ on the deposition zone of the paper chip. Subsequently, to start the reaction, the solution containing the sample, previously added with platinum nanoparticles coated with citrate, is dripped onto the deposition zone. The possible presence of Hg²⁺ ions in the water samples rapidly inhibits the peroxidase activity of Pt nanoparticles, so that there is no generation of the blue color due to TMB oxidation.

Similarly, the article "Citrate-capped platinum nanoparticle as a smart probe for ultrasensitive mercury sensing", G.W. Wu et al., Analytical Chemistry, 86, 10955-10960 (2014), by the same authors and with content corresponding to the patent application CN 104267026 A, describes a method for *in situ* analysis of mercury (II) in water similar to the previous one, but carried out in liquid phase rather than on a solid support. The method involves conducting the analysis at 45 °C, a condition that compromises the applicability of the method in a simple and point-of-care kit.

Finally, the article "Visual observation of the mercury-stimulated peroxidase mimetic activity of gold nanoparticles", Y.J. Long et al., Chem. Commun., 2011, 47, 11939-11941, describes a method for detecting mercury based on the increase in the ability of gold nanoparticles to catalyze the TMB oxidation reaction by H₂O₂ as a result of their reaction with mercury.

The applicability of these methods on to real samples is demonstrated using the spike technique, which consists in adding a known amount of contaminant into the matrix of a real sample (in this case, tap water).

This technique is widespread, but it does not take into account the fact that, in the case of Hg²⁺, a sample in which a known amount of standard is added (typically Hg(NO₃)₂ or HgCl₂) immediately before the analysis is not really representative of the situation of a real contaminated sample. In fact, the Hg²⁺ ion in nature establishes equilibria with the other species present in solution particularly in fresh water, characterized by a pH close to neutrality and a concentration of chlorides of about 1-2 mM, mercury speciation is predominated by the soluble species Hg(OH)₂ and HgOHCl. This phenomenon is not visible by carrying out a normal spike experiment of Hg²⁺ standards in real samples; it is instead necessary to let the samples equilibrate for several hours, so as to obtain a sample that is actually representative of the real situation. The species that are formed are not reduced by species derived from carboxylic acids (such as the citrate ion) as easily as Hg²⁺_{(aq)}, and this is an obstacle to the applicability of all the previously described methods that involve a reduction of mercury on to real samples.

As a consequence of the phenomenon of mercury speciation in water, for example, the method described in the cited article by G.W. Wu *et al.* and in the patent application CN 104267026 A is not effective in detecting the presence of traces of mercury in real samples.

The tests reported in these documents are performed on samples produced through the spike technique. As described above, these samples are not representative of the real samples, because they do not have the characteristic speciation equilibria. The same tests, performed on samples equilibrated for a few hours, wherein mercury speciation becomes representative of that in the real samples, are not able to reveal mercury.

Moreover, none of the methods known in the literature which involve the reduction of Hg²⁺ take into account another feature of the real samples: the presence of active chlorine in the form of hypochlorite/hypochlorous acid as a disinfectant. In Western countries, it is established by law that in drinking water a residual concentration of disinfectant, mainly chlorine, has to be present; the recommended value is 0.5 mg/L, and it is estimated to typically have values between 0.2 and 1 mg/L. These concentrations of chlorine are able to interfere with the redox systems involved in the *in situ* detection techniques described above. It is therefore necessary to include a strategy for the removal of chlorine, avoiding that this additional step, and any reagents used, interfere with the subsequent determination mechanism. The main techniques for chlorine removal consist of physical methods, such as active carbon filters or UV irradiation. The filters also retain some ionic species, modifying the original matrix, while UV treatments are difficult to apply in the perspective of a simple, rapid and inexpensive *in situ* detection.

The currently known colorimetric methods are therefore inadequate for the determination of mercury contamination in real samples of drinking water.

Therefore, there is currently no valid alternative to traditional instrumental techniques, that allows the simple and rapid detection of mercury in drinking water, overcoming the obstacles due to the matrix, such as mercury speciation and the presence of residual active chlorine.

The object of the present invention is to provide a method that overcomes the issues of the prior art, allowing the analysis of real water samples, avoiding the matrix interferences of the known methods.

### SUMMARY OF THE INVENTION

This object is achieved with the present invention that, in a first aspect thereof, relates to a method for the colorimetric determination of mercury in water, comprising the following steps:
a) withdrawing a sample of water;
b) adding to the sample of water nanoparticles of a noble metal selected from Au, Pd, and Pt with a diameter of between 1 and 100 nm, in a concentration of between 1 fM and 100 nM, obtaining a first mixture;
c) letting the first mixture obtained in step b) incubate for at least 5 minutes, obtaining a pretreated sample;
d) adding to the pretreated sample obtained in step c) a chromogenic agent, H₂O₂, and an acid buffer at a pH of between 4 and 6, obtaining a second mixture; and
e) detecting whether or not a chromatic variation of said second mixture occurs at a preset time, which depends on the reagents employed, after obtaining said second mixture,
characterized in that:
in step b), for the preparation of said first mixture, an acidic pH buffer and a reducing agent having a redox potential of between + 0.34 V and + 0.85 V are further added to the sample of water.

The method of the present invention is defined in claim 1.

In a second aspect thereof, the invention relates to a portable kit for performing the aforementioned test for the determination of mercury in water, comprising the following elements:
1) a transparent container for water sampling;
2) containers containing pre-dosed amounts of the reagents used in step b) of the method described above; and
3) containers containing pre-dosed amounts of the reagents used in step d) of the method described above.

The kit of the invention is defined in claim 12.

### BRIEF DESCRIPTION OF THE FIGURES

- Fig. 1 shows a photograph of a sample of water containing mercury and a sample of water not containing the element, on both of which the method of the invention was carried out;
- Fig. 2 shows the time course of the absorbance at 652 nm of two samples containing mercury, on one of which a test according to the invention was carried out, while on the other a test under similar conditions was carried out, but without using an acidic pH buffer;
- Fig. 3 shows the time course of the absorbance at 652 nm of two samples containing mercury, on one of which a test according to the invention was carried out, while on the other a test under similar conditions was carried out, but without using a reducing agent.

### DETAILED DESCRIPTION OF THE INVENTION

In the description and claims, the following terms are to be intended as specified:
- "nanoenzyme" is a nanometric material, generally a metal nanoparticle, able to show catalytic activity as an enzyme;
- "chromogenic agent" is a chemical compound able to transform itself from colorless into a coloring substance upon the creation of pre-established conditions;
- "mild reducing agent" is a chemical substance able to yield electrons to an oxidizing agent, and having a redox potential of between + 0,34 V and + 0,85 V.

The inventors observed that the preconditioning of the water sample to be analyzed with a mild reducing agent as defined above and the acid buffer allows to eliminate the interferences in the analysis by compounds containing chlorine, which are normally used for the disinfection of drinking water. Furthermore, preconditioning overcomes the problem of mercury speciation in water, and involves an increase in the efficiency of the amalgam reaction, which is preparatory to the colorimetric determination step, without however interfering with the redox reaction involving the chromogenic agent.

In step a) of the method, the water sample is taken using common sampling techniques for water analysis, at a temperature of between 4 and 40 °C, preferably between 10 and 30 °C. Preferably the water sample is a sample of drinking water taken, for example, from a common tap.

In step b), an acidic pH buffer, a mild reducing agent and nanoparticles of a noble metal selected from Au, Pd and Pt are added to the sample of step a); the nanoparticles are added to the sample in an amount such as to obtain a concentration of between 1 fM and 100 nM (concentration intended as the number of moles of nanoparticles per liter), obtaining a first mixture. Among the nanoparticles, Pt ones are preferred, because it was observed that they are those giving the best reactivity, and therefore sensitivity, of the method.

The nanoparticles useful for the method of the invention are Au, Pd and, preferably, Pt nanoparticles stabilized with citrate, and have a diameter of between 1 and 100 nm, preferably between 2 and 30 nm, and even more preferably of about 20 nm. The production of metal nanoparticles is known, and may be carried out, for example, according to the general method described in the article "Monodisperse Platinum Nanospheres with Adjustable Diameters from 10 to 100 nm: Synthesis and Distinct Optical Properties", N.C. Bigall et al., Nano Lett., 2008, 8 (12), pp. 4588-4592. Preferably, for the objectives of the present invention, nanoparticles are produced according to a modified technique with respect to that of the mentioned article, which uses as starting material "seeds" of platinum, the preparation of which is described in bullet point (1) on p. 4589 of the article by Bigall *et* al. According to this modified procedure, if one wants to obtain, for example, 20 nm diameter monodisperse nanoparticles, 3 mL of "seeds" solution are added to 87 mL of distilled water (for example, MilliQ) together with 108 µl of a 0.5 M solution of H₂PtCl₆ and 1.5 mL of a solution containing 1.25% sodium citrate and 1% L-ascorbic acid (% m/v concentrations, *i.e.* expressed in g of solute per 100 mL of solution); the temperature is slowly brought to 100 °C, and the reaction mixture is kept at this temperature under stirring for one hour, and finally let naturally cool to room temperature. The nanoparticles thus obtained are then washed with a 2 mM solution of sodium citrate on 50 K Amicon filters to remove any contaminants.

The acid buffer is added in such an amount to obtain a concentration in the range of 1 to 100 mM, and a pH of between 4 and 7 in the pretreated sample of step b) of the method; acid/salt pairs useful for the purposes of the invention are selected from those corresponding to 2-(N-morpholino)ethansulfonic (abbreviated with the acronym MES), acetic, citric, and phosphoric acid; the salt of the buffer pair is generally the salt of an alkaline metal, preferably sodium or potassium.

Said mild reducing reducing agent is added in such an amount that its concentration in the pretreated sample is of between 500 nM and 5 mM, and may be of inorganic or organic nature. Examples of suitable inorganic reducing agents are: metallic copper, salts containing iodide anion, and salts containing iron(II) cation; examples of suitable organic reducing agents are ascorbic acid, ethylenediaminetetraacetic acid (EDTA), cytochromes, hydroquinones, and photosystems, whose redox potentials can be modulated by slightly interfering *ad hoc* with the pH of the system. Preferably the organic mild reducing agent used is ascorbic acid.

The pretreatment step of step b) of the method of the invention allows to convert the various species that characterize the mercury speciation present in the real samples of fresh water in Hg²⁺_{(aq)}. In this way, the analyte is made entirely available for the next colorimetric determination step.

With the acidification of the sample, it is in fact possible to interfere with the equilibria among the species present in solution, thus shifting the following reactions to the left:

Hg²⁺ + 2H₂O ↔ Hg(OH)_{2(aq)} + 2H⁺

Hg²⁺ + Cl⁻ + H₂O ↔ HgOHCl_{(aq)} + H⁺

At the same time, a double effect is exerted by the reducing agent present in the system: elimination of chlorine interference and increase in the efficiency of the reduction of Hg²⁺_{(aq)} to metallic mercury and the consequent formation of amalgam.

During this step, the nanoparticle/analyte interaction takes place, which exploits the high redox potential of mercury and its tendency, in its oxidation state 0, to form amalgams with other metals.

The presence of a reducing agent in the amalgam formation reaction is in contrast with the state of the art, since it is known that reducing agents strongly inhibit the colorimetric reaction catalyzed by platinum nanoparticles. The invention thus achieves a compromise between the positive actions of the reducing agent (contrasting chlorine and increasing the efficiency of the amalgam reaction) and the non-interference on the peroxidase activity of nanoparticles.

In step c), the first mixture is left to rest ("incubation") for a period of about 5 minutes, to allow complete formation of the amalgam, obtaining a pretreated sample.

In step d) of the invention, an acid buffer (such as MES, acetate, citrate, phosphate), a chromogenic substrate such as 3,3',5,5'-tetramethylbenzidine (TMB) or 2,2'-azino-bis (3-ethylbenzothiazolin-6-sulphonic) acid (ABTS) and H₂O₂ are added to the pretreated sample thus obtaining a second mixture. Preferably, said acid buffer is an acetate buffer and said chromogenic agent is TMB. In this case, the reaction intermediate that is formed during the radical oxidation of TMB by H₂O₂ is metastable and, thanks to the high delocalization of its electrons, has an intense blue color.

A suitable choice of the reaction conditions allows to obtain a concentration of oxidized substrate such that, in a determined period of time useful for diagnostic purposes, is capable of conferring a color to the solution; for example, using an acetate buffer at pH 4.6, Pt nanoparticles with a diameter of 20 nm, and TMB as a chromogenic agent, the final reaction product appears light blue.

Finally, in step e) of the method, the colorimetric test result is observed. In the absence of mercury, a variation in the appearance of the sample is observed; for example, in the case of TMB, a light blue color noticeably appears within 5-7 minutes. The color change of the suspension from transparent to colored is due to the redox reaction, catalyzed by the Pt nanoparticles, between the chromogenic agent and hydrogen peroxide. In fact, the chromogenic agent in its oxidized form is responsible for the color variation, easily observable with the naked eye, of the entire sample; the color develops clearly in a time of about 10 minutes.

Conversely, in the presence of an amount of mercury above the limits of the law, the sample remains completely transparent since mercury deposition on the metal nanoparticle surface inhibits the catalysis of the redox reaction between the chromogenic agent and H₂O₂; as a consequence, the chromogenic agent remains in its reduced form and no coloring is observed in the sample. In fact, the assay has been optimized to be a threshold assay: concentrations above the limits of the law give such an inhibition to ensure that the sample remains transparent for at least 10 minutes, while the non-contaminated ones become light blue during a period of time of between 5 and 7 minutes.

In a second aspect thereof, the invention relates to a kit for carrying out the method described above. This kit comprises a graduated dropper or syringe for sampling the water and, subsequently, the first mixture, a container containing pre-dosed amounts of the reagents used in step b) of the method, and a transparent container containing pre-dosed amounts of the reagents used in step d) of the method described above.

The dropper for sampling the water is preferably graduated, giving an indication of a predetermined withdrawal volume, such that the addition of the compounds present in the other containers of the kit brings the concentrations of the same to values useful for the method described above.

As an example, a kit for carrying out the method may comprise:
1) a graduated dropper or syringe for sampling the water, graduated for the withdrawal of 1.8 mL of water and for the subsequent withdrawal of 0.1 mL of the first mixture obtained in the method of the invention;
2) a container A having a storage capability of 2 mL and containing the following reagents in pre-dosed amounts for the preparation of the first mixture (sample pre-conditioning step):
   ▪ 0.196 mL of a citric acid/sodium citrate buffer at 0.1 M citrate ion concentration and a pH of 5.0;
   ▪ 0.020 mL of a 125 pM suspension of Pt nanoparticles with a 20 nm diameter in a 2 mM trisodium citrate aqueous solution;
   ▪ 0.020 mL of a 5 mM ascorbic acid solution;
3) a transparent container **B** for the chromogenic reaction having a 1 mL storage capability and containing the following reagents in pre-dosed amounts for the preparation of the second mixture:
   ▪ 0.600 mL of an acetic acid/sodium acetate buffer at 16.7 mM acetate ion concentration and a pH of 4.5;
   ▪ 0.100 mL of a 5 mM TMB solution
   ▪ 0.200 mL of a 1 M H₂O₂ solution.

In an alternative embodiment of the kit, the ascorbic acid present in the container **A** is in a lyophilized form, deposited on the bottom of the same container, in an amount of 1.76 × 10⁻⁵ g (amount equivalent to that derived from concentration and volume indicated above); this condition increases its stability over time.

The three reagents present inside the container **A** and the three reagents present inside the container **B** are kept separate until use, for example by means of a tank cap system which releases the solutions upon screwing.

The above exemplified kit may also be used by a person who is not skilled in the chemical analysis field, according to the simple procedure which involves the following steps:
- immediately after mixing the reagents in container **A,** the pre-measured volume of sample (1.8 mL - the dropper is filled up to a notch marked with an intuitive indication, for example "1", and emptied completely into container **A)** is added to them;
- after having waited for the incubation time (step c of the method of the invention), the reagents present in container B are mixed and, immediately after this, the pre-measured volume of mixture from container **A** (0.1 mL - the dropper must be filled up to a notch marked with an intuitive indication, for example "2", and emptied completely into the container **B**) is added to this mixture.
The method of the invention and the kit for carrying it out can therefore be applied to *in situ* determination of mercury in real water samples, allowing to discriminate, in a few minutes and with the naked eye, between samples of uncontaminated water, within the range of concentrations relevant to the law, and mercurycontaminated water. Method and kit do not require the use of any dangerous reagents and are therefore also suitable for the execution by users who are inexperienced and not trained in the field of analytical chemistry.

The invention will be further illustrated by the following examples.

### EXAMPLE 1

This example refers to a test for detecting mercury in samples of water from Genoa water network according to the method of the invention.

A sample of monodisperse PtNPs with a 20 nm diameter, prepared according to the modified method of Bigall *et al.* described in the text, was diluted in 2 mM trisodium citrate and brought to a final concentration of 125 pM.

9.8 µl of 100 mM citrate buffer pH 5.0, 1 µl of PtNPs suspension prepared as described above, and 1 µl of 5 mM ascorbic acid solution were added to 88.2 µl of tap water. After 5 minutes, 900 µl of a solution consisting of 100 µl of 100 mM acetate buffer pH 4.5, 100 µl of 5 mM TMB, 200 µl of 1 M H₂O₂ and 500 µl bidistilled water were added to it. Over a period of between 4 and 7 minutes the suspension visibly turns to light blue.

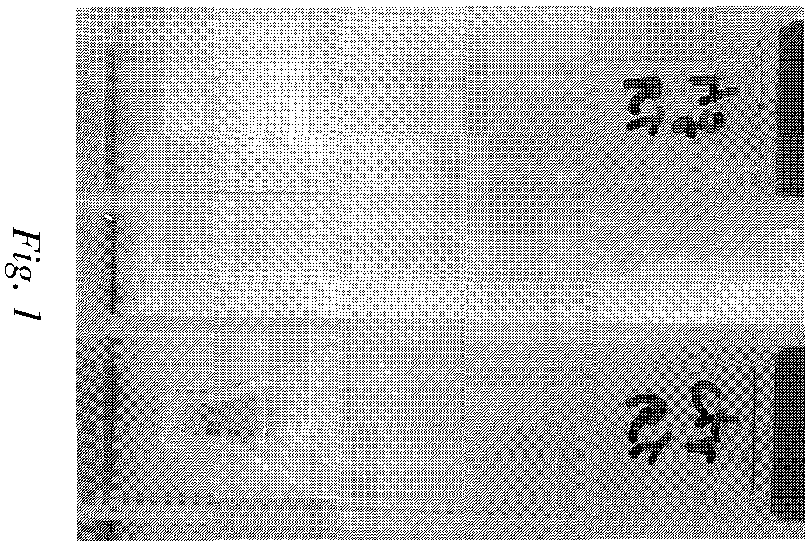

For comparison, the test was conducted in parallel, under the same conditions described above, but adding to the water withdrawn from the water network a volume of a solution of Hg²⁺ in 10% HNOs (ICP standard) so as to obtain a nominal concentration of the element of 80 nM. The sample was let to equilibrate for 24 hours before performing the test, to allow the distribution of mercury among the various species that characterize its speciation. 10 minutes after the end of the tests, the two water samples were photographed; the result is shown in Figure 1: on the left the contaminated sample with a colorless appearance is shown, while on the right the non-intentionally contaminated sample with a gray appearance (corresponding to light blue in the original color image) is shown.

Both reactions are carried out at uncontrolled room temperature and all reagents were equilibrated at room temperature.

### EXAMPLE 2 (COMPARATIVE)

This example refers to a test for detecting mercury in samples of water from Genoa water network according to the method described in the article by G.W. Wu *et al.* cited in the text (content corresponding to CN 104267026 A).

A suspension of Pt nanoparticle coated with citrate ion was prepared as described on page 10956 of the article.

100 µl of this suspension were added to 400 µl of tap water; after two minutes, the sample was diluted with 2.3 mL of ultrapure water, then 1 mL of 2 M H₂O₂ and 200 µL of 16 mM TMB were added to it, and the system was allowed to react at 45 °C for 10 minutes.

The solution remained completely colorless, because the chromogenic reaction cannot take place under the described conditions, *i.e.* in the absence of acid catalysis.

For comparison, the test was conducted in parallel, under the same conditions described above, but adding to the water withdrawn from the water network a volume of a solution of Hg²⁺ in 10% HNOs (ICP standard) so as to obtain a nominal concentration of the element of 80 nM. The sample was let to equilibrate for 24 hours before performing the test, to allow the distribution of mercury among the various species that characterize its speciation. Again, the solution remained completely colorless, because the chromogenic reaction cannot take place under the described conditions, *i.e.* in the absence of acid catalysis. The method is not able to discriminate the presence of mercury in the sample of tap water.

### EXAMPLE 3 (COMPARATIVE)

This example refers to a test for detecting mercury in samples of water from Genoa water network according to the method of the invention. It is aimed at highlighting the effect of the pretreatment with an acid buffer in step b).

A sample of monodispersed PtNPs with a 20 nm diameter, prepared according to the modified method of Bigall *et al.* described in the text, was diluted in 2 mM trisodium citrate and brought to a final concentration of 125 pM.

A volume of a solution of Hg²⁺ in 10% HNOs (ICP standard) was added to a sample of tap water so as to obtain a nominal concentration of the element of 80 nM. The sample was let to equilibrate for 24 hours before performing the test, to allow the distribution of mercury among the various species that characterize its speciation.

9.8 µl of bidistilled water, 1 µl of PtNPs suspension prepared as described above, and 1 µl of 5 mM ascorbic acid solution were added to 88.2 µl of this equilibrated sample. After 5 minutes, 900 µl of a solution consisting of 100 µl of 100 mM acetate buffer pH 4.5, 100 µl of 5 mM TMB, 200 µl of 1 M H₂O₂ and 500 µl of bidistilled water were added to it. The mercury present in this sample did not inhibit the nanoparticles, and after 5 minutes the suspension started to turn to light blue. The development of the chromogenic intermediate was monitored by measuring the increase in absorbance at 652 nm as a function of time.

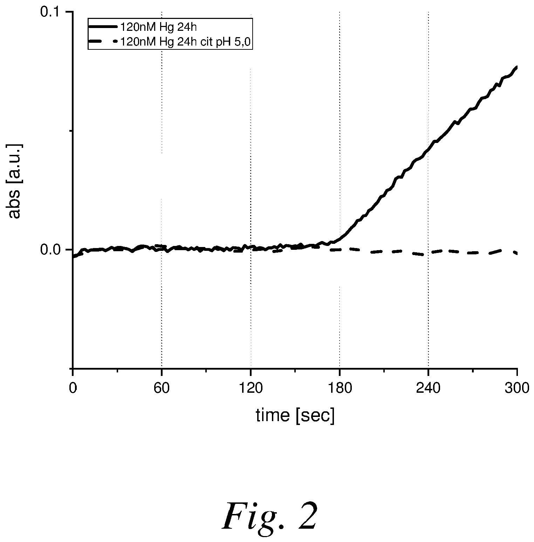

For comparison, the test was conducted in parallel, under the same conditions described above, but adding 9.8 µl of 100 mM citrate buffer pH 5.0 to the equilibrated sample instead of bidistilled water. The mercury present in this sample inhibited the nanoparticles, and after 5 minutes the sample was still completely colorless. The development of the chromogenic intermediate was monitored by measuring the increase in absorbance at 652 nm as a function of time. The result is shown in Figure 2: the solid line describes the time course of the absorbance of the sample in which the mercury did not inhibit the nanoparticles due to its speciation; the dotted line describes the time course of the absorbance of the sample in which the nanoparticles were inhibited by mercury.

Both reactions are carried out at uncontrolled room temperature and all reagents were equilibrated at room temperature.

### EXAMPLE 4 (COMPARATIVE)

This example refers to a test for detecting mercury in samples of water from Genoa water network according to the method of the invention. It is aimed at highlighting the effect of the pretreatment with a mild reducing agent in step b).

A sample of monodispersed PtNPs with a 20 nm diameter, prepared according to the modified method of Bigall *et al.* described in the text, was diluted in 2 mM trisodium citrate and brought to a final concentration of 125 pM.

A volume of a solution of Hg²⁺ in 10% HNOs (ICP standard) was added to a sample of tap water so as to obtain a nominal concentration of the element of 80 nM. The sample was let to equilibrate for 24 hours before performing the test, to allow the distribution of mercury among the various species that characterize its speciation.

9.8 µl of 100 mM citrate buffer pH 5.0, 1 µl of PtNPs suspension prepared as described above, and 1 µl of bidistilled water were added to 88.2 µl of this equilibrated sample. After 5 minutes, 900 µl of a solution consisting of 100 µl of 100 mM acetate buffer pH 4.5, 100 µl of 5 mM TMB, 200 µl of 1 M H₂O₂ and 500 µl of bidistilled water were added to it. The mercury present in this sample did not inhibit the nanoparticles, and almost immediately the suspension started to turn to light blue. The development of the chromogenic intermediate was monitored by measuring the increase in absorbance at 652 nm as a function of time.

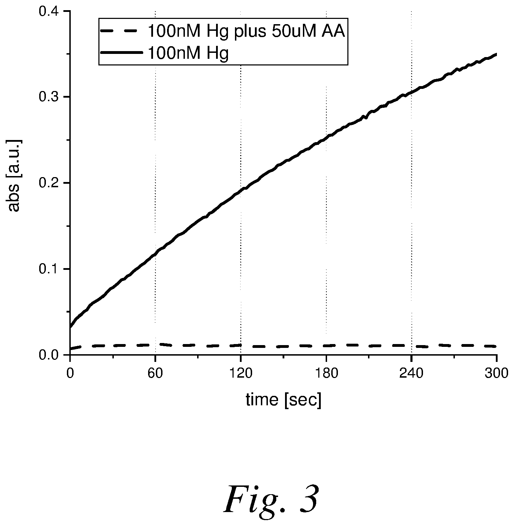

For comparison, the test was conducted in parallel, under the same conditions described above, but adding 1 µl of 5 mM ascorbic acid solution to the equilibrated sample instead of bidistilled water. The mercury present in this sample inhibited the nanoparticles and after 5 minutes the sample was still completely colorless. The development of the chromogenic intermediate was monitored by measuring the increase in absorbance at 652 nm as a function of time. The result is shown in Figure 3: the solid line describes the time course of the absorbance of the sample in which the mercury did not inhibit the nanoparticles due to the absence of ascorbic acid; the dotted line describes the time course of the absorbance of the sample in which the nanoparticles were inhibited by the mercury reduced by ascorbic acid.

Both reactions are carried out at uncontrolled room temperature and all reagents were equilibrated at room temperature.

The currently known colorimetric methods are therefore inadequate for the determination of mercury contamination in real samples of drinking water.

Comparative examples 3 and 4 demonstrate that the methods known from the literature, therefore without the use of an acid buffer and a mild reducing agent in step b), allow to evaluate the presence of mercury in samples produced *ad hoc* and tested within a short time after their production, but not in real samples.

## Claims

1. A method for the colorimetric determination of mercury in water, comprising the following steps:
a) withdrawing a sample of water;
b) adding to the sample of water nanoparticles of a noble metal selected from Au, Pd and Pt with a diameter of between 1 and 100 nm in a concentration of between 1 fM and 100 nM, obtaining a first mixture;
c) letting the first mixture obtained in step b) incubate for at least 5 minutes, obtaining a pretreated sample;
d) adding to the pretreated sample obtained in step c) a chromogenic agent, H₂O₂, and an acid buffer at a pH of between 4 and 6, obtaining a second mixture; and
e) detecting whether or not a chromatic variation of said second mixture occurs at a preset time, which depends on the reagents employed, after obtaining said second mixture,
**characterized in that**:
in step b), for the preparation of said first mixture, an acidic pH buffer and a reducing agent having a redox potential of between + 0.34 V and + 0.85 V are further added to the sample of water.

2. Method according to claim 1, wherein in step a) the sample of water is taken at a temperature of between 4 and 40 °C, preferably between 10 and 30 °C.

3. Method according to any one of claims 1 or 2, wherein the nanoparticles employed in step b) are stabilized with citrate and have a diameter of between 2 and 30 nm, preferably of about 20 nm.

4. Method according to any one of the preceding claims, wherein Pt nanoparticles are employed.

5. Method according to any one of the preceding claims, wherein the acid buffer employed in step b) has a pH of between 4 and 7 and a concentration of between 1 and 100 mM.

6. Method according to claim 5, wherein said acid buffer is comprised of an acid and a corresponding salt pair, wherein the acid is selected from 2-(N-morpholino)ethanesulfonic acid (MES), acetic acid, citric acid, and phosphoric acid.

7. Method according to any one of the preceding claims, wherein in step b) the reducing agent is employed in such an amount that its concentration thereof in the first mixture is of between 500 nM and 5 mM.

8. Method according to any one of the preceding claims, wherein the reducing agent employed in step b) is selected from metallic copper, salts containing iodide anion, salts containing iron(II) cation, ascorbic acid, ethylenediaminetetraacetic acid (EDTA), cytochromes, hydroquinones and photosystems.

9. Method according to any one of the preceding claims, wherein the chromogenic agent employed in step d) is selected from 3,3',5,5'-tetramethylbenzidine (TMB) and 2,2'-azino-bis(3-ethylbenzothiazoline-6-sulfonic) acid (ABTS).

10. Method according to any one of the preceding claims, wherein the acid buffer employed in step d) consists of an acid and corresponding salt pair, wherein the acid is selected from 2-(N-morpholino)ethanesulfonic acid (MES), acetic acid, citric acid, and phosphoric acid.

11. Method according to any one of the preceding claims, wherein in step b) monodispersed Pt nanoparticles with a diameter of 20 nm are employed, and in step d) an acetic acid/sodium acetate buffer at pH 4.6 and TMB as a chromogenic agent are employed, and the detection of the possible chromatic variation in step e) is carried out 10 minutes after the completion of step d).

12. Kit for carrying out the method of any one of claims 1 to 11, comprising:
- a graduated dropper or syringe;
- a container comprising an acidic pH buffer, a reducing agent having a redox potential of between + 0.34 V and + 0.85 V, and nanoparticles of a noble metal selected from Au, Pd and Pt with a diameter of between 1 and 100 nm in a concentration of between 1 fM and 100 nM; and
- a transparent container comprising pre-dosed amounts of a chromogenic agent, H2O2, and an acid buffer at a pH of between 4 e 6.

13. Kit according to claim 12, comprising the following elements:
1) a graduated dropper or syringe having a notch for the withdrawal of 1.8 mL of water and a notch for the subsequent withdrawal of 0.1 mL of the first mixture obtained in step b) of the method of the invention;
2) a container **A** having a storage capability of 2 mL, and containing the following reagents in pre-dosed amounts:
▪ 0.196 mL of a citric acid/sodium citrate buffer at 0.1 M citrate ion concentration and a pH of 5.0;
▪ 0.020 mL of a 125 pM suspension of Pt nanoparticles with a 20 nm diameter in a 2 mM trisodium citrate aqueous solution;
▪ 0.020 mL of a 5 mM ascorbic acid solution or, alternatively, an equivalent amount of ascorbic acid in solid form on the bottom of the container;
3) a transparent container **B** having a storage capability of 1 mL and containing the following reagents in pre-dosed amounts:
▪ 0.600 mL of an acetic acid/sodium acetate buffer at 16.7 mM acetate ion concentration and a pH of 4.5;
▪ 0.100 mL of a 5 mM TMB solution;
▪ 0.200 mL of a 1 M H₂O₂ solution.

## Patentansprüche

1. Verfahren zur kolorimetrischen Bestimmung von Quecksilber in Wasser, welches die folgenden Schritte umfasst:
a) Entnehmen einer Wasserprobe;
b) Zugeben von Nanopartikeln eines aus Au, Pd und Pt ausgewählten Edelmetalls mit einem Durchmesser zwischen 1 und 100 nm in einer Konzentration zwischen 1 fM und 100 nM zu der Wasserprobe, wobei ein erstes Gemisch erhalten wird;
c) Inkubierenlassen des in Schritt b) erhaltenen Gemisches für mindestens 5 Minuten, wobei eine vorbehandelte Probe erhalten wird;
d) Zugeben eines chromogenen Mittels, von H₂O₂ und eines Säurepuffers bei einem pH zwischen 4 und 6 zu der in Schritt c) erhaltenen vorbehandelten Probe, wobei ein zweites Gemisch erhalten wird; und
e) Nachweisen, ob nach dem Erhalten des zweiten Gemisches zu einer vorgegebenen Zeit, die von den eingesetzten Reagenzien abhängt, eine farbliche Veränderung des zweiten Gemisches eintritt oder nicht,
**dadurch gekennzeichnet, dass**:
in Schritt b) zur Herstellung des ersten Gemisches zudem ein saurer pH-Puffer und ein Reduktionsmittel, das ein Redoxpotential zwischen +0,34 V und +0,85 V aufweist, zu der Wasserprobe zugegeben werden.

2. Verfahren nach Anspruch 1, wobei die Wasserprobe in Schritt a) bei einer Temperatur zwischen 4 und 40°C genommen wird, bevorzugt zwischen 10 und 30°C.

3. Verfahren nach einem der Ansprüche 1 oder 2, wobei die in Schritt b) eingesetzten Nanopartikel mit Citrat stabilisiert werden und einen Durchmesser zwischen 2 und 30 nm, bevorzugt von etwa 20 nm, aufweisen.

4. Verfahren nach einem der vorangehenden Ansprüche, wobei Pt-Nanopartikel eingesetzt werden.

5. Verfahren nach einem der vorangehenden Ansprüche, wobei der in Schritt b) eingesetzte Säurepuffer einen pH zwischen 4 und 7 und eine Konzentration zwischen 1 und 100 mM aufweist.

6. Verfahren nach Anspruch 5, wobei der Säurepuffer eine Säure und ein entsprechendes Salzpaar umfasst, wobei die Säure aus 2-(N-Morpholino)ethansulfonsäure (MES), Essigsäure, Zitronensäure und Phosphorsäure ausgewählt ist.

7. Verfahren nach einem der vorangehenden Ansprüche, wobei in Schritt b) das Reduktionsmittel in einer solchen Menge eingesetzt wird, dass seine Konzentration in dem ersten Gemisch zwischen 500 nM und 5 mM liegt.

8. Verfahren nach einem der vorangehenden Ansprüche, wobei das in Schritt b) eingesetzte Reduktionsmittel aus metallischem Kupfer, Salzen, die ein lodid-Anion enthalten, Salzen, die ein Eisen(II)-Kation enthalten, Ascorbinsäure, Ethylendiamintetraessigsäure (EDTA), Cytochromen, Hydrochinonen und Photosystemen ausgewählt ist.

9. Verfahren nach einem der vorangehenden Ansprüche, wobei das in Schritt d) eingesetzte chromogene Mittel aus 3,3',5,5'-Tetramethylbenzidin (TMB) und 2,2'-Azino-bis(3-ethylbenzothiazolin-6-sulfonsäure) (ABTS) ausgewählt ist.

10. Verfahren nach einem der vorangehenden Ansprüche, wobei der in Schritt d) eingesetzte Säurepuffer aus einer Säure und einem entsprechenden Salzpaar besteht, wobei die Säure aus 2-(N-Morpholino)ethansulfonsäure (MES), Essigsäure, Zitronensäure und Phosphorsäure ausgewählt ist.

11. Verfahren nach einem der vorangehenden Ansprüche, wobei in Schritt b) monodisperse Pt-Nanopartikel mit einem Durchmesser von 20 nm eingesetzt werden, und in Schritt d) ein Essigsäure/Natriumacetat-Puffer bei pH 4,6 und TMB als ein chromogenes Mittel eingesetzt werden, und der Nachweis der möglichen farblichen Veränderung in Schritt e) 10 Minuten nach dem Abschluss des Schrittes d) durchgeführt wird.

12. Kit zum Ausführen des Verfahrens nach einem der Ansprüche 1 bis 11, welches umfasst:
- eine skalierte Pipette oder Spritze;
- einen Behälter, der
einen sauren pH-Puffer,
ein Reduktionsmittel, das ein Redoxpotential zwischen +0,34 V und +0,85 V aufweist, und Nanopartikel eines aus Au, Pd und Pt ausgewählten Edelmetalls mit einem Durchmesser zwischen 1 und 100 nm in einer Konzentration zwischen 1 fM und 100 nM umfasst; und
- einen transparenten Behälter, der vordosierte Mengen
eines chromogenen Mittels,
von H₂O₂ und
eines Säurepuffers bei einem pH zwischen 4 und 6 umfasst.

13. Kit nach Anspruch 12, welches die folgenden Teile umfasst:
1) eine skalierte Pipette oder Spritze, die eine Einkerbung für die Entnahme von 1,8 mL Wasser und eine Einkerbung für die anschließende Entnahme von 0,1 mL des in Schritt b) des erfindungsgemäßen Verfahrens erhaltenen ersten Gemisches aufweist;
2) einen Behälter **A,** der eine Aufnahmekapazität von 2 mL aufweist und die folgenden Reagenzien in vordosierten Mengen enthält:
▪ 0,196 mL eines Zitronensäure/Natriumcitrat-Puffers bei 0,1 M Citrat-Ionen-Konzentration und einem pH von 5,0;
▪ 0,020 mL einer 125 pM Suspension von Pt-Nanopartikeln mit einem Durchmesser von 20 nm in einer 2 mM wässrigen Trinatriumcitrat-Lösung;
▪ 0,020 mL einer 5 mM Ascorbinsäure-Lösung oder, alternativ, eine äquivalente Menge an Ascorbinsäure in fester Form am Boden des Behälters;
3) einen transparenten Behälter **B,** der eine Aufnahmekapazität von 1 mL aufweist und die folgenden Reagenzien in vordosierten Mengen enthält:
▪ 0,600 mL eines Essigsäure/Natriumacetat-Puffers bei 16,7 mM Acetat-Ionen-Konzentration und einem pH von 4,5;
▪ 0,100 mL einer 5 mM TMB-Lösung;
▪ 0,200 mL einer 1 M H₂O₂-Lösung.

## Revendications

1. Procédé pour la détermination colorimétrique du mercure dans l'eau, comprenant les étapes suivantes :
a) le prélèvement d'un échantillon d'eau ;
b) l'ajout à l'échantillon d'eau de nanoparticules d'un métal noble choisi parmi Au, Pd et Pt avec un diamètre compris entre 1 et 100 nm à une concentration comprise entre 1 fM et 100 nM, en obtenant un premier mélange ;
c) le fait de laisser incuber le premier mélange obtenu à l'étape b) pendant au moins 5 minutes, en obtenant un échantillon prétraité ;
d) l'ajout à l'échantillon prétraité obtenu à l'étape c) d'un agent chromogène, H2O2, et un tampon acide à un pH compris entre 4 et 6, en obtenant un second mélange ; et
e) détecter si une variation chromatique dudit second mélange se produit ou non à un moment prédéterminé, qui dépend des réactifs employés, après l'obtention dudit second mélange,
**caractérisé en ce que** :
à l'étape b), pour la préparation dudit premier mélange, un tampon de pH acide et un agent réducteur ayant un potentiel redox compris entre + 0,34 V et + 0,85 V sont en outre ajoutés à l'échantillon d'eau.

2. Procédé selon la revendication 1, dans lequel, à l'étape a), l'échantillon d'eau est prélevé à une température comprise entre 4 et 40°C, de préférence entre 10 et 30°C.

3. Procédé selon l'une quelconque des revendications 1 ou 2, dans lequel les nanoparticules utilisées à l'étape b) sont stabilisées avec du citrate et ont un diamètre compris entre 2 et 30 nm, de préférence d'environ 20 nm.

4. Procédé selon l'une quelconque des revendications précédentes, dans lequel des nanoparticules de Pt sont utilisées.

5. Procédé selon l'une quelconque des revendications précédentes, dans lequel le tampon acide utilisé à l'étape b) a un pH compris entre 4 et 7 et une concentration comprise entre 1 et 100 mM.

6. Procédé selon la revendication 5, dans lequel ledit tampon acide est constitué d'une paire d'un acide et d'un sel correspondant, dans lequel l'acide est choisi parmi l'acide 2-(N-morpholino)éthanesulfonique (MES), l'acide acétique, l'acide citrique et l'acide phosphorique.

7. Procédé selon l'une quelconque des revendications précédentes, dans lequel, à l'étape b), l'agent réducteur est employé dans une quantité telle que sa concentration dans le premier mélange soit comprise entre 500 nM et 5 mM.

8. Procédé selon l'une quelconque des revendications précédentes, dans lequel l'agent réducteur utilisé à l'étape b) est choisi parmi le cuivre métallique, les sels contenant un anion d'iodure, les sels contenant un cation de fer(II), l'acide ascorbique, l'acide éthylènediamine-tétraacétique (EDTA), les cytochromes, les hydroquinones et les photosystèmes.

9. Procédé selon l'une quelconque des revendications précédentes, dans lequel l'agent chromogène utilisé à l'étape d) est choisi parmi la 3,3',5,5'-tétraméthylbenzidine (TMB) et l'acide 2,2'-azino-bis(3-éthylbenzothiazoline-6-sulfonique) (ABTS).

10. Procédé selon l'une quelconque des revendications précédentes, dans lequel le tampon acide utilisé à l'étape d) consiste en une paire d'acide et de sel correspondant, dans lequel l'acide est choisi parmi l'acide 2-(N-morpholino)éthanesulfonique (MES), l'acide acétique, l'acide citrique et l'acide phosphorique.

11. Procédé selon l'une quelconque des revendications précédentes, dans lequel, à l'étape b), des nanoparticules de Pt monodispersées avec un diamètre de 20 nm sont utilisées, et à l'étape d), un tampon acide acétique/acétate de sodium à pH 4,6 et de la TMB comme agent chromogène sont utilisés, et la détection de la variation chromatique éventuelle à l'étape e) est effectuée 10 minutes après la fin de l'étape d).

12. Kit pour la réalisation du procédé selon l'une quelconque des revendications 1 à 11, comprenant
- un compte-gouttes ou une seringue gradués ;
- un récipient comprenant
un beurre au pH acide,
un agent réducteur ayant un potentiel redox compris entre + 0,34 V et + 0,85 V, et
des nanoparticules d'un métal noble choisi parmi Au, Pd et Pt avec un diamètre compris entre 1 et 100 nm dans une concentration comprise entre 1 fM et 100 nM ; et
- un récipient transparent comprenant des quantités pré-dosées de
un agent chromogène,
H2O2, et
un beurre acide à un pH compris entre 4 et 6.

13. Kit selon la revendication 12, comprenant les éléments suivants :
1) un compte-gouttes ou une seringue gradués ayant une encoche pour le prélèvement de 1,8 ml d'eau et une encoche pour le prélèvement ultérieur de 0,1 ml du premier mélange obtenu à l'étape b) du procédé de l'invention ;
2) un récipient **A** ayant une capacité de stockage de 2 ml, et contenant les réactifs suivants en quantités pré-dosées :
• 0,196 ml d'un tampon acide citrique/citrate de sodium à une concentration d'ions de citrate de 0,1 M et à un pH de 5,0 ;
• 0,020 ml d'une suspension de 125 pM de nanoparticules de Pt avec un diamètre de 20 nm dans une solution aqueuse de citrate trisodique de 2 mM ;
• 0,020 ml d'une solution d'acide ascorbique 5 mM ou, en variante, une quantité équivalente d'acide ascorbique sous forme solide sur le fond du récipient ;
3) un récipient transparent **B** ayant une capacité de stockage de 1 ml et contenant les réactifs suivants en quantités pré-dosées :
• 0,600 ml d'un tampon acide acétique/acétate de sodium à une concentration d'ions d'acétate de 16,7 mM et un pH de 4,5 ;
• 0,100 ml d'une solution de TMB de 5 mM ;
• 0,200 ml d'une solution 1 M de H2O2.
